# EUROPEAN PATENT APPLICATION

(11) **EP 4 537 807 A1**
(43) Date of publication of application: **16.04.2025**
(21) Application number: 22945914.4
(22) Date of filing: 07.06.2022
(51) Int. Cl.: A61K 8/65, A61Q 7/00, A61K 38/16, A61P 17/14

(54) **HAIR-GROWING OR HAIR-FOSTERING AGENT BASED ON KERATIN FINE PARTICLES**

(71) Applicant: Myqtech Inc., Tsukuba-shi, Ibaraki 305-8573 (JP)
(72) Inventor: YAMAMOTO, Yohei, Tsukuba-shi, Ibaraki 305-8573 (JP); HEAH, Wey Yih, Tsukuba-shi, Ibaraki 305-8573 (JP); YAMAGISHI, Hiroshi, Tsukuba-shi, Ibaraki 305-8573 (JP); ISODA, Hiroko, Tsukuba-shi, Ibaraki 305-8572 (JP)
(74) Representative: Mewburn Ellis LLP
(86) International application number: PCT/JP2022/023554
(87) International publication number: WO 2023/238416

(57) **Abstract**

The present invention provides a hair growing and hair fostering agent demonstrating high hair growing and hair fostering effects with no or low side effects. Specifically, the present invention provides a hair growing or hair fostering agent comprising keratin microparticles as a hair growing or hair fostering active ingredient.

## Description

### Technical Field

The present invention relates to a hair growing or hair fostering agent comprising keratin microparticles. The present invention also relates to a cosmetic or pharmaceutical composition comprising the hair fostering or hair growing agent.

### Background Art

Representative chemical agents that have been known to stimulate hair fostering and hair growing are Minoxidil, Finasteride and Dutasteride. Minoxidil, which has the vasodilation effect, promotes blood circulation of capillary vessels under the scalp when applied and supplies lots of nutrients to follicle cells, whereby the hair growing and hair fostering effects are expressed. However, vasodilation causes blood reduced pressure and can detrimentally affect the heart and other organs, and in some cases poses a critical condition leading to a fatal consequence. Finasteride and Dutasteride are observed to have hair loss prevention and hair thickening effect by suppressing the hair removal effect caused by dihydrotestosterone (DHT), but liver dysfunction has been reported as a serious side effect.

On the other hand, keratin is a protein, which is the principal component for hair, claw, feather, scale, hoof, wool and horn of animals such as birds, mammals, and reptiles. Keratin is non-toxic, biodegradable with excellent cell viability and well biocompatible with live cells. For this reason, studies and applications on the use of keratin typically involve fields of drug development, pharmaceutical product, cosmetic product, food product, agriculture and biotechnology. The purpose of keratin application also includes, in addition to drug delivery, applications such as cell adhesion, cell proliferation, tissue scaffold, tissue regeneration, cell proliferation, wound healing, external injury, bioactive surface, and medical implant device (e.g., Non Patent Literatures 1 and 2). Keratin can be formed into multiple shapes in the forms of porous foam, sponge, mat, film, sheet, gel, microfiber, microparticles (microspheres), nanoparticles (nanospheres), and bulk material (e.g., Non Patent Literature 2).

### Citation List

### Non Patent Literature

Non Patent Literature 1: A. Aluigi, et al., Int. J. Biol. Macromol., 2007, 41, 266-273
Non Patent Literature 2: K. Katoh, et al., Biomaterials, 2004, 25, 2265-2272
Non Patent Literature 3: Seong Yeong An, et al., Research Square, archive, https://www.researchsquare.com/article/rs-101358/v1
Non Patent Literature 4: S. Hu et al., Sci Adv. 2020, 6(30): eaba1685

### Summary of Invention

### Technical Problem

Various effects of keratin have been reported such as hair growth and hair fostering effects when keratin itself is subcutaneously injected (Non-Patent Literature 3). Additionally, keratin in 3D spheroid of follicle dermal papilla cells expresses the hair growing effect (Non-Patent Literature 4). However, both effects are highly invasive involving pains of subcutaneous injection. For this reason, there has been a demand on techniques capable of providing hair growing and hair fostering effects of keratin using a formulation such as a noninvasive application preparation that does not involve pains.

Further, the development of a hair growing and hair fostering agent that exhibit high hair growing and hair fostering effects with no or few toxicity and side effects has been still in demand, although there are a wide variety of hair growing and hair fostering agents available.

### Solution to Problem

The present inventors have found that keratin microparticles exhibit higher hair growing and hair fostering effects when applied than a typical keratin aqueous dispersion and have found that keratin microparticles, which are non-toxic to the human body, are applicable to hair growth and hair fostering, skin care and health care, whereby the present invention has come to completion.

For example, the present invention encompasses the following embodiments.
[1] A hair growing or hair fostering agent comprising keratin microparticles as a hair growing or hair fostering active ingredient.
[2] The hair growing or hair fostering agent according to [1], wherein the keratin microparticles have sizes of 0.01 to 100 µm.
[3] The hair growing or hair fostering agent according to [1] or [2], wherein the keratin microparticles are in the form of an aqueous solution, an aqueous dispersion, or a hydrogel.
[4] The hair growing or hair fostering agent according to any of [1] to [3], wherein the keratin comprises human keratin, wool-derived keratin, or partially sulfonated or ionized water-soluble keratin.
[5] A cosmetic composition comprising the hair growing or hair fostering agent according to any of [1] to [4] and a cosmetically acceptable excipient.
[6] A pharmaceutical composition comprising the hair growing or hair fostering agent according to any of [1] to [4] and a pharmaceutically acceptable excipient.
[7] The pharmaceutical composition according to [6], which is an external preparation for skin.
[8] A method for growing hair or fostering hair in a subject, comprising applying the hair growing or hair fostering agent according to any of [1] to [4] or keratin microparticles to the subject.
[9] A method for treating or preventing alopecia in a subject, comprising applying the hair growing or hair fostering agent according to any of [1] to [4] or keratin microparticles to the subject.
[10] The method according to [8] or [9], wherein the application to the subject comprises the application to skin of the subject.
[11] The method according to [9], wherein the alopecia comprises alopecia areata, androgenetic and female androgenetic alopecia, alopecia caused by systemic diseases (such as chronic thyroiditis, iron-deficiency anemia, and connective tissue disease), drug-induced alopecia (alopecia caused by anticancer agent and the like) and the like.
[12] Keratin microparticles for use in a hair growing or hair fostering agent.
[13] Keratin microparticles for use in growing hair or fostering hair in a subject.

### Advantageous Effects of Invention

The present invention provides a hair growing or hair fostering agent that exhibits high hair growing and hair fostering effects. Keratin microparticles as the active ingredient are non-toxic to animals including human, and thus may particularly be effective as a cosmetic product and a medicine. Keratin microparticles are also biodegradable and have a low environmental impact.

### Brief Description of Drawings

Figure 1 is photographs showing the hair growing effect of keratin microparticles on mice.
Figure 2 is a graph of principal component analysis (PCA) and a Venn diagram showing changes in gene expressions (up regulation and down regulation) treated with 1% Minoxidil, a 1% keratin aqueous dispersion (1% keratin) or a 1% keratin microparticle suspension (1% keratin microparticles).
Figure 3 is scatter plots showing changes in gene expressions (up regulation and down regulation) treated with a 1% keratin aqueous dispersion (1% keratin) or a 1% keratin microparticle suspension (1% keratin microparticle).
Figure 4 is heat maps of hierarchical clustering of changes in gene expressions (up regulation and down regulation) treated with a 1% keratin aqueous dispersion (1% keratin) or a 1% keratin microparticle suspension (1% keratin microparticle).
Figure 5 is a graph showing up-regulated gene ontology (GO) treated with an aqueous dispersion of keratin microparticles or keratin powder.
Figure 6A is a graph showing up-regulated gene pathways treated with an aqueous dispersion of keratin microparticles or keratin powder.
Figure 6B is a graph showing down-regulated gene pathways treated with an aqueous dispersion of keratin microparticles or keratin powder.
Figure 7 shows a Venn diagram and a heat map of hierarchical clustering of the genes changed when treated with a 1% keratin aqueous dispersion and a 1% keratin microparticle suspension.
Figure 8 is graphs showing fold changes in gene expressions in biological processes by keratin microparticles.
Figure 9A is graphs showing results obtained by microarray validations (Ctnnb1, Notch1) by RT-PCR.
Figure 9B is graphs showing results obtained by microarray validations (DUX, Cdh11) by RT-PCR.
Figure 9C is graphs showing results obtained by microarray validations (Lnk, K14) by RT-PCR.
Figure 10 is photographs showing the adsorption of a keratin microparticle suspension on a skin surface after applied to the skin surface.
Figure 11 is photographs showing the adsorption of a keratin microparticle suspension on a skin surface after applied to the skin surface.
Figure 12 is photographs showing before and after a keratin microparticle suspension dropped on a glass and dried. A: keratin spherical microparticle ( microparticle in the dry state); B: the state of keratin spherical microparticles dispersed in water (swollen spherical gel); C: the state of keratin spherical microparticles dispersed in water dropped on a glass and dried (dried disk-like structure).

### Description of Embodiments

Hereinafter, the present invention is described in detail.

The present invention relates to a hair growing or hair fostering agent comprising keratin microparticles as a hair growing or hair fostering active ingredient. According to the present invention, the "hair growing" and "hair fostering" mean to promote the growth of hair (e.g., hair on the head, eyebrow, eyelash, and beard) or hair growth, to increase hair volume (the number, thickness and the like), to reduce or suppress hair removal, and to improve the condition of hair growth area (e.g., inflammatory condition) and the like.

According to the present invention, the "keratin" can be naturally derived or synthesized as long as it is a protein, which is the principal component of hair, claw, feather, scale, hoof, wool and horn of animals and can be used as a cosmetic product or a medicine. Keratin, as long as it is originally derived from a naturally-occurring organism or environment, can be pretreated such as purification and extraction, or a recombinant product based on the sequence information thereof. Additionally, keratin, as long as it has the function equal to the naturallyderived keratin, can be a variant or a homologue thereof, or chemically modified. Average molecular weight (number average molecular weight) of keratin may be, for example, about 100 to 100000, and preferably about 200 to 40000. In a preferable embodiment, keratin may contain human keratin, wool- or feather-derived keratin, or water-soluble keratin in which these keratins are partially sulfonated or ionized. Keratin may be used singly, or two or more kinds of keratins may be used in combination.

Keratin may be commonly prepared by a person skilled in the art, or a commercial product may be purchased.

According to the present invention, keratin may be used in the form of microparticles having particle sizes of nano-to-micrometer scales. According to the present invention, the term "microparticles" refers to spherical or approximately spherical particles having particle sizes of about 0.01 to 500 µm, but they do not always need to maintain the shape of spherical as long as they are microscopic. In an embodiment, the particle sizes of keratin microparticles may be preferably 0.1 to 100 µm, and more preferably 1 to 10 µm. In another embodiment, at least 90% of keratin microparticles may have the particle sizes of 0.05 to 100 µm, preferably the particle sizes of 0.1 to 20 µm, and more preferably the particle sizes of 1 to 10 µm.

Keratin microparticles can be prepared by any known method in the technical field in the art. For example, a preparation method of microparticles using keratin regenerated from wool (F. Cilurzo, et al., Polym. Adv. Technol., 2013, 24, 1025-1028), a preparation method of keratin microparticles using keratin extracted from human hair (Y. Srisuwan and P. Srihanam, Orient. J. Chem., 2019, 35, 1112-1116), and a preparation method of a microcapsule based on wool keratin (H. Rajabinejad, et al., Ultrason. Sonochem., 2018, 40, 527-532) may be used.

In a preferable embodiment, a keratin solution may be supplied to a sprayer, an air gun or an air brush using an adjusted compressed gas, directly sprayed to a poor solvent (e.g., ethyl acetate) to allow the keratin mist to precipitate in the poor solvent, then allowed the suspension to stand for 6 to 24 hours to cause precipitation, whereby the precipitated keratin microparticles may be collected.

By the above, keratin microparticles in the solution may be formed. The formed keratin microparticles may be centrifuged and collected, then washed in a suitable solvent, thereby removing an unnecessary reagent.

The hair growing or hair fostering agent according to the present invention may contain keratin microparticles as the active ingredient for growing hair or fostering hair. Keratin microparticles may be used directly, but preferably used by being dissolved, dispersed or suspended in a solvent. The solvent that may be used is not particularly limited but may preferably be a cosmetically or pharmaceutically acceptable solvent. For example, keratin microparticles may be dissolved, dispersed or suspended in a solvent such as water, a salt solution (e.g., saline). Thus, keratin microparticles can be present in the form of, for example, an aqueous solution, an aqueous dispersion, or a hydrogel.

The hair growing or hair fostering agent according to the present invention may exhibit a hair growing and/or hair fostering effect, which may be a faster and/or thicker hair growing and/or hair fostering effect compared with, for example, a case with no treatment. Preferably, the hair growing or hair fostering agent according to the present invention may have no or few side effects. Examples of the side effect may include inflammation (itchiness, rash, red flare and the like), initial hair loss, perspiration, chill, palpitation, dizziness, headache, abdominal pain, liver dysfunction (nausea, poor appetite and the like), jaundice, decreased libido, and excessive hair growth (hirsutism).

The hair growing or hair fostering agent according to the present invention may not particularly limit the subject to which it is used (a subject being treated). For example, the present agent can be applied to mammals such as human, primates (monkey, chimpanzee and the like), livestock (cow, horse, pig and the like), companion animals (dog, cat and the like), and laboratory animals (mouse, rat, monkey and the like). For example, animal subjects having an area onto which hair growth or hair fostering is desired, particularly human, may be preferable as the subject.

The hair growing or hair fostering agent according to the present invention can be used as a composition. For example, keratin microparticles may be mixed with an appropriate carrier or diluted or suspended in a carrier to form the composition. Examples of the appropriate carrier may include salt solutions (e.g., saline), lactose, dextrose, sucrose, sorbitol, mannitol, xylitol, erythritol, maltitol, starch, Acacia gum, alginate, gelatin, calcium phosphate, calcium silicate, cellulose, methyl cellulose, microcrystalline cellulose, polyvinyl pyrrolidone, water, methyl hydroxybenzoate, propyl hydroxybenzoate, talc, magnesium stearate and mineral oils. Additionally, the composition may comprise an excipient, a surfactant, a dispersant, a buffer, a preservative, a solubilizer, a stabilizer, a tonicity agent and/or the like which are commonly used. Further, other components or compositions can also be mixed or blended in keratin microparticles. Examples of other components may include, but not limited thereto, other known hair growing or hair fostering components (ascorbic acid, Ginkgo biloba extract, Stemoxydine, Minoxidil, Finasteride, Dutasteride and the like) and other effective components (hair active ingredients, moisturizer, softener, anti-inflammatory agent, minerals, amino acids and the like).

The content of the hair growing or hair fostering agent (keratin microparticles) in the composition may vary depending on the form of the composition, the subject to which the agent is used, the purpose of application and the like, and a person skilled in the art can suitably determine in consideration of these. For example, the composition can comprise 0.01 wt% to 50 wt%, preferably 0.05 wt% to 20 wt%, of keratin microparticles.

In an embodiment, the hair growing or hair fostering agent (keratin microparticles) according to the present invention may be blended in a cosmetic product and used as a cosmetic composition. The cosmetic product into which the agent is blended may not be particularly limited as long as the cosmetic product is applicable to an area (e.g., hair on the head and beard) onto which the hair growth or hair fostering is desired. Examples may include hair care products (shampoo, treatment, hair spray, hair pack, hair liquid, hair tonic and the like), lotion, milky lotion, and cream. The cosmetic composition can comprise a cosmetically acceptable excipient or carrier such as a bulking agent, a binder, a wetting agent, a lubricant, a surfactant, a dispersant, a buffer, a pH regulator, a preservative, a solubilizer, an antiseptic, an absorption enhancer, a stabilizer, a tonicity agent, and a fragrance. Additionally, the cosmetic composition can comprise other known hair growing or hair fostering active components (ascorbic acid, Ginkgo biloba extract, Stemoxydine, Minoxidil, Finasteride, Dutasteride and the like) and other components (hair active ingredients, moisturizer, softener, anti-inflammatory agent, minerals, amino acids and the like). The cosmetic composition can be produced by, for example, a method such as mixing by a routine method depending on the form thereof.

In another embodiment, the hair growing or hair fostering agent (keratin microparticles) according to the present invention may be blended in a medicine and used as a pharmaceutical composition (e.g., an external preparation for skin). According to the present description, the "medicine" encompasses quasi drugs. The dosage form of the pharmaceutical composition can be an appropriate dosage form depending on the subject or purpose to which the composition is applied and can be preferably external preparations for skin such as an ointment, a cream agent, a lotion agent (a liquid agent), a gel agent, an aerosol agent, and a transdermal patch, and can also be other dosage forms suitable for parenteral administration (e.g., intracutaneous administration and subcutaneous administration). The pharmaceutical composition can comprise a pharmaceutically acceptable excipient or carrier such as a bulking agent, a binder, a wetting agent, a lubricant, a surfactant, a dispersant, a buffer, a pH regulator, a preservative, a solubilizer, an antiseptic, an absorption enhancer, a stabilizer and a tonicity agent. Additionally, the pharmaceutical composition can comprise other known hair growing or hair fostering active components (Minoxidil, Finasteride, Dutasteride and the like) and other components (moisturizer, softener, anti-inflammatory agent, minerals, amino acids and the like). The pharmaceutical composition can be produced by, for example, a method such as mixing, applying, and spraying by a routine method depending on the form thereof.

The pharmaceutical composition can be used as an external preparation for skin to treat or prevent a disease or a symptom at an area onto which hair growth or hair fostering is desired. The pharmaceutical composition can be used to treat or prevent a disease or a symptom such as alopecia areata, androgenetic and female androgenetic alopecia, alopecia caused by systemic diseases (such as chronic thyroiditis, iron-deficiency anemia, and connective tissue disease (collagen disease)), drug-induced alopecia (alopecia caused by anticancer agent and the like) and the like.

The amount, frequency and period of the composition used can be suitably determined depending on the amount of keratin microparticles comprised, the hair volume and condition of a subject to which the composition is used. The composition, when applied to skin, can be generally used in about 1 µg to 50 mg/cm² at one time, 1 to 3 times a day, once every 2 days, once every 3 days, once a week, for 1 week to 3 months, further 1 month to 1 year or more, and, if necessary, over a period of several years to several decades.

The present invention also relates to a method for growing hair or fostering hair in a subject comprising applying (e.g., applying to skin) the hair growing or hair fostering agent (keratin microparticles) according to the present invention to the subject. Additionally, the present invention relates to a method for treating or preventing alopecia in a subject, comprising applying the hair growing or hair fostering agent (keratin microparticles) according to the present invention to the subject (e.g., topical application such as applying to skin, subcutaneous or intracutaneous administration).

Further, keratin microparticles in combination with cells can be used, for example, as a material for regenerative medicine. For example, a keratin microparticle suspension may be applied to 3D spheroid and the 3D spheroid may be administered or transplanted to a subject, whereby the keratin effect can be demonstrated on the subject. Alternatively, cells (e.g., hair matrix cell) may be administered with keratin microparticles, thereby exhibiting the hair fostering and hair growing effects of keratin.

### Examples

Hereinafter, the present invention will be described in further detail in reference to examples and drawings. The following examples do not intend to limit the present invention.

### [Example 1] Preparation of keratin microparticles

Keratin microparticles (also referred to as keratin spherical microparticles) were prepared as follows. 1 to 2 mL of a water-soluble keratin aqueous solution (concentration 0.1 to 2 wt%, partially sulfonated, TCI) was supplied to an ultrasonic mist generator, and the generated keratin mist was added dropwise to 100 mL of a poor solvent (e.g., EtOAc) filled in a 200 mL beaker. The obtained suspension was allowed to stand for 6 to 24 hours, and the generated keratin microparticles were precipitated. The precipitated keratin microparticles were filtered, collected and washed several times with the poor solvent. Finally, the keratin microparticles were dried at 25°C and further dried in a vacuum oven (40°C, -0.1 MPa) to remove residual solvent.

### [Example 2] Verification of hair growing effect of keratin microparticles on mice

In the present example, the hair growing effect of keratin microparticles on mice was investigated. The experiment was approved by Institutional Animal Care and Use Committee, University of Tsukuba (No. 17-060), and the procedure was carried out in conformity with the "Guiding principles for the care and use of animals in the field of physiological sciences" approved by the board of Physiological Society of Japan.

Six-week-old male C57BL/6 mice (Charles River Japan Inc.) were kept individually in a cage, acclimated for 1 week, and then randomly assigned to experiment groups (each group n=5) of a 1% Minoxidil treated group, a 1% keratin treated group, a 1% keratin microparticles treated group, and a milli-Q water treated group (control group). The mice were anesthetized with Isoflurane (Wako Pure Chemical Industries, Ltd.) and then telogen hair at the dorsal region of the mice was shaved using a hair clipper to induce the growth phase. Then, each mouse was topically applied every day for 4 weeks with the 1% Minoxidil (Tokyo Chemical Industry Co., Ltd.), 1% keratin aqueous dispersion (Tokyo Chemical Industry Co., Ltd.), 1% keratin microparticle suspension (the keratin microparticles prepared in Example 1 were suspended in water), or milli-Q water. The shaved dorsal region was observed and photographed everyday starting day 14 after treated.

In the end of treatment period, the mice were slaughtered by cervical dislocation. The skin collected from the treated area was divided into 4 parts, washed with phosphate buffered saline (PBS), then immediately immersed in liquid nitrogen and maintained at -80°C. Using ImageJ processing program (National Institutes of Health, Bethesda, USA), the hair regrowth at the treated area was measured.

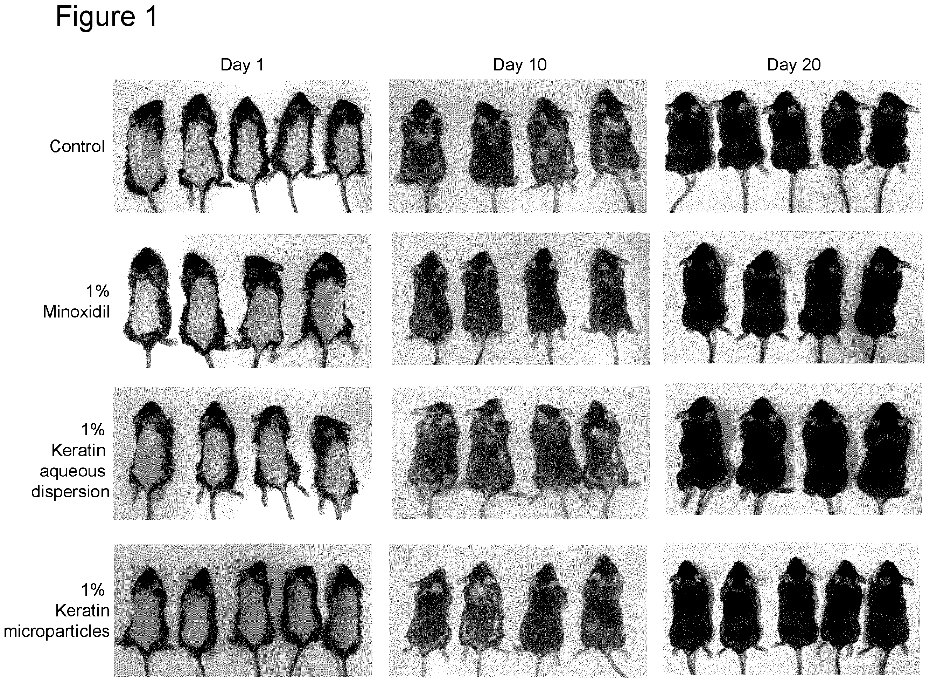

The results are shown in Figure 1. The mice to which the keratin aqueous dispersion was applied were verified to have hair grown to the same level as the mice to which nothing was applied (control). On the other hand, the mice to which the keratin microparticles were applied demonstrated the hair regrowth phenotype to the same level as the mice to which Minoxidil was applied at about the same time (day 10 after application), thereby showing hair growth promoting effect of accelerated hair cycle when compared with the control and the keratin aqueous dispersion.

### [Example 3] Verification of hair growing effect of keratin microparticles on mice

In the present example, the skin cells of mice treated in Example 2 were genetically analyzed using DNA microarray.

RNA used as the template was extracted from the skin tissues collected from the treated area of the mouse dorsal region in Example 2 using ISOGEN reagent (NIPPON GENE CO., LTD.) in accordance with the instruction of the manufacturer. The extracted total RNA was amplified and labeled. Subsequently, the labeled fragmented RNA was hybridized with Affymetrix mouse Array strips (Affymetrix). GeneChip (Mouse Genome 430 2.0 Array) was washed, stained, scanned using Affymetrix GeneAtlas Imaging Station, thereby obtaining mRNA expressions of various genes from mouse genome.

Then, gene ontology, biological processes and fold changes in gene expressions (2-fold change, control vs 1% Minoxidil, 1% keratin aqueous dispersion, 1% keratin microparticle) were analyzed using Transcriptome Analysis Console (TAC) software (version 4.0.1) and bioinformatics resource 6.8 which is database for annotation, visualization, and integrated discovery (DAVID). Average signal log 2 (control vs 1% Minoxidil, 1% keratin aqueous dispersion, 1% keratin microparticles) was subjected to hierarchical clustering using Euclidean distance and average chain algorithm of TIGR Mev version 3.0.3 software (The Institute for Genomic Research, MD, USA).

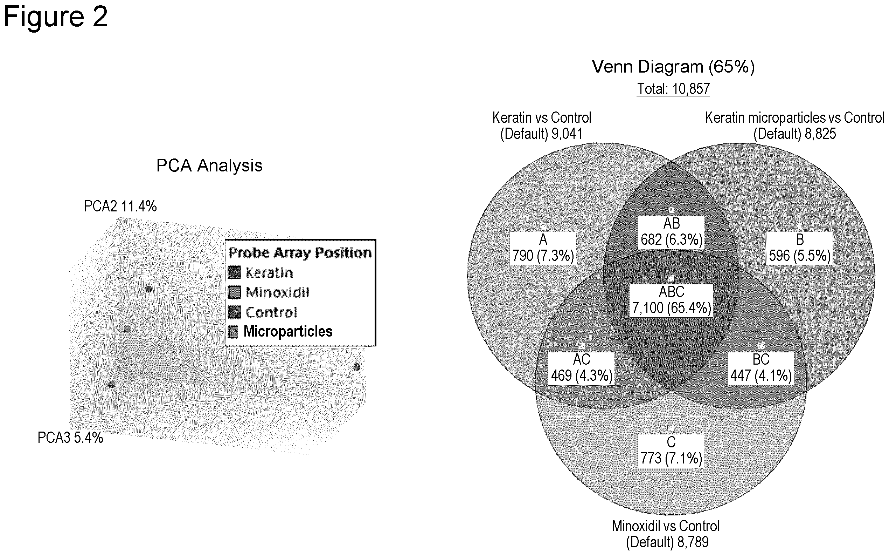

The results obtained are shown in Figures 2 to 9. The results are shown in mean ± standard deviation (SD) (n=2, respectively). Statistical analysis was carried out using Student's t-test when comparing two sets of values (control vs 1% Minoxidil, 1% keratin aqueous dispersion, 1% keratin microparticles). P value ≤ 0.05 was considered significant. For evaluating the significance standard between treated groups *in vivo,* ANOVA (p-value (Condition pair) < 0.05) was carried out. In Figures 2 to 9, the 1% keratin aqueous dispersion is denoted simply as Keratin.

Figure 2 is a graph showing principal component analysis (PCA) and Venn Diagram, and differentially expressed genes of the up-regulated (>2) or down-regulated (<-2) genes compared with the control by respective treatment (1% Minoxidil, 1% keratin aqueous dispersion, 1% keratin microparticles). The results shown in Figure 2 revealed that gene expressions different from the control are found in all treatments. Total number of differentially expressed genes, the number of up-regulated genes and the number of down-regulated genes are collectively shown in Table 1 below. The numbers of expressed genes sufficient for the analysis are shown.

**[Table 1]**

| | Differentially expressed genes | | |
|---|---|---|---|
| | Total number | Up-regulated | Down-regulated |
| Keratin microparticles vs control | 11602 | 10470 | 1132 |
| Keratin aqueous dispersion vs control | 12260 | 11018 | 1242 |
| Minoxidil vs control | 13471 | 11913 | 1558 |

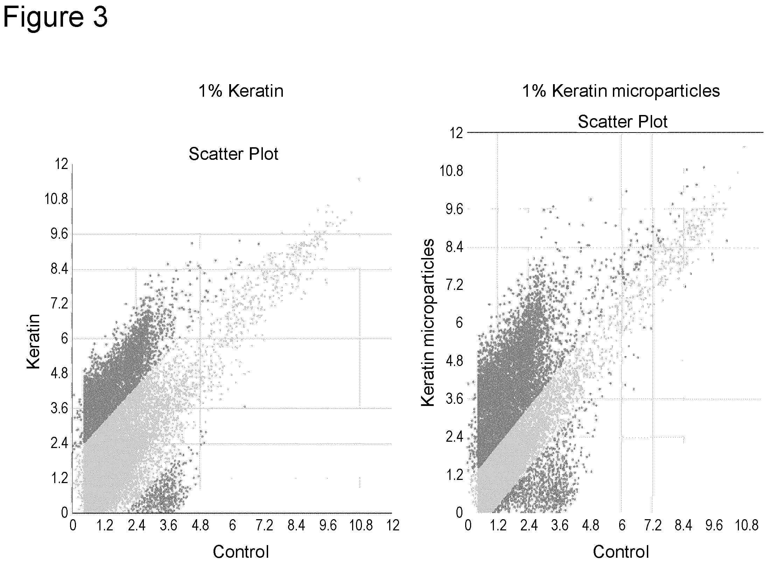

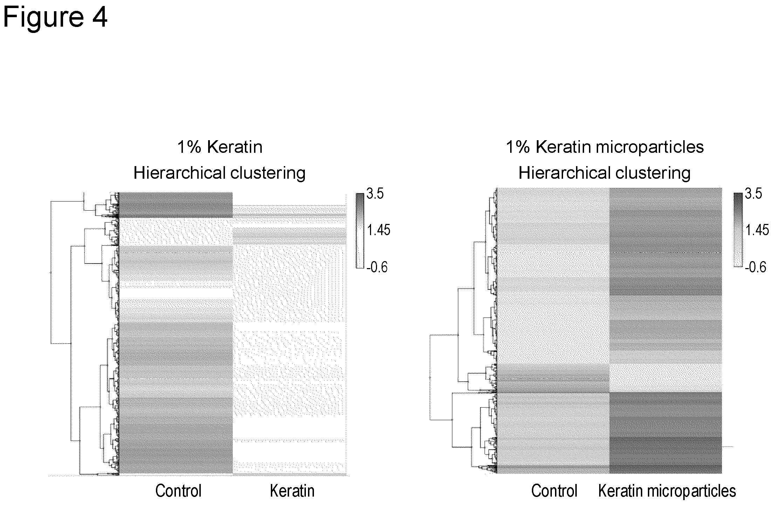

Subsequently, changes in gene expression (up regulation and down regulation) compared with the control when treating with the 1% keratin aqueous dispersion and 1% keratin microparticle suspension were analyzed by Scatter Plot (Figure 3) and Hierarchical clustering (Figure 4). Of the investigated gene total number 45077, up-regulated genes were 11018 (89.87%) and down-regulated genes were 1242 (10.13%) among the genes meeting the criteria 12260 (27.2%) in the case of treating with the 1% keratin aqueous dispersion, whereas up-regulated genes were 8403 (89.52%) and down-regulated genes were 984 (10.48%) among the genes meeting the criteria 9387 (20.82%) in the case of treating with the 1% keratin microparticle suspension. The Scatter Plots shown in Figure 3 reveal that the gene expression changes are different by the 1% keratin aqueous dispersion and the 1% keratin microparticles compared with the control. Additionally, the heat maps of Hierarchical clustering shown in Figure 4 reveal that different gene expression patterns are obtained by the keratin aqueous dispersion and the keratin microparticles compared with the control.

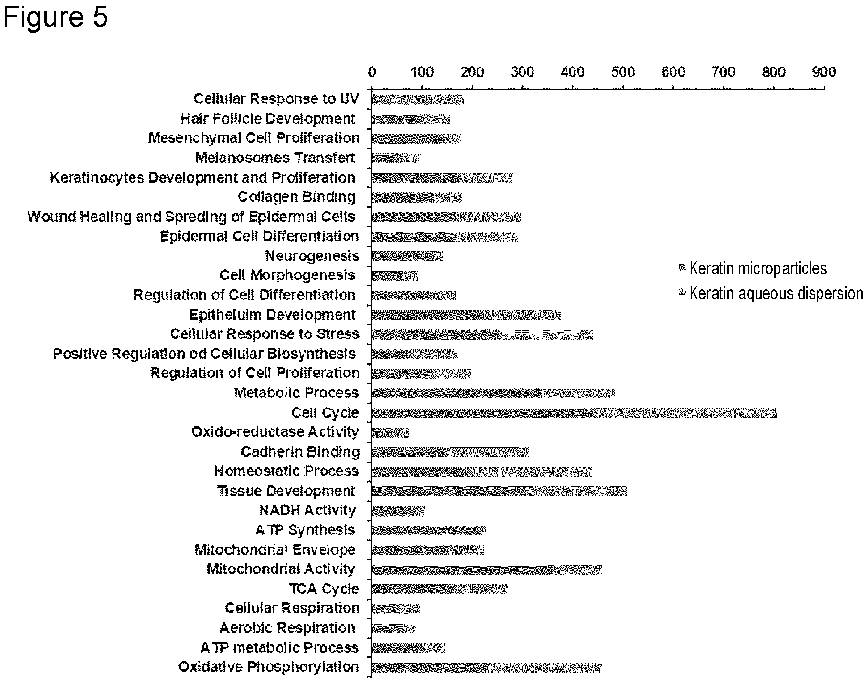

Figure 5 shows up-regulated gene ontology (GO) compared with the control by the treatment with the keratin microparticles or the keratin aqueous dispersion. For example, the up-regulated genes compared with the control are associated with Epithelium Development, Cellular Response to Stress, Metabolic Process, Cell Cycle, Homeostatic process, Tissue Development, Mitochondrial Activity, and Oxidative Phosphorylation, and particularly keratin microparticles contributed to up regulation of the gene associated with Cell Cycle. In other words, the keratin aqueous dispersion and the keratin microparticles up-regulate the gene ontology of hair fostering, development of the epidermis, and cell adhesion, and the effect thereof was more remarkable in the keratin microparticles treated group.

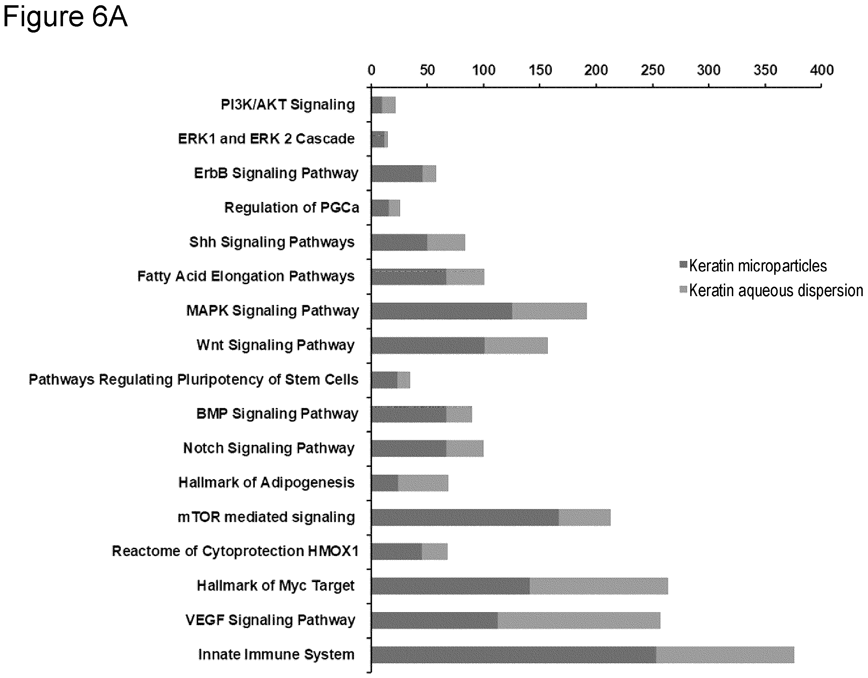

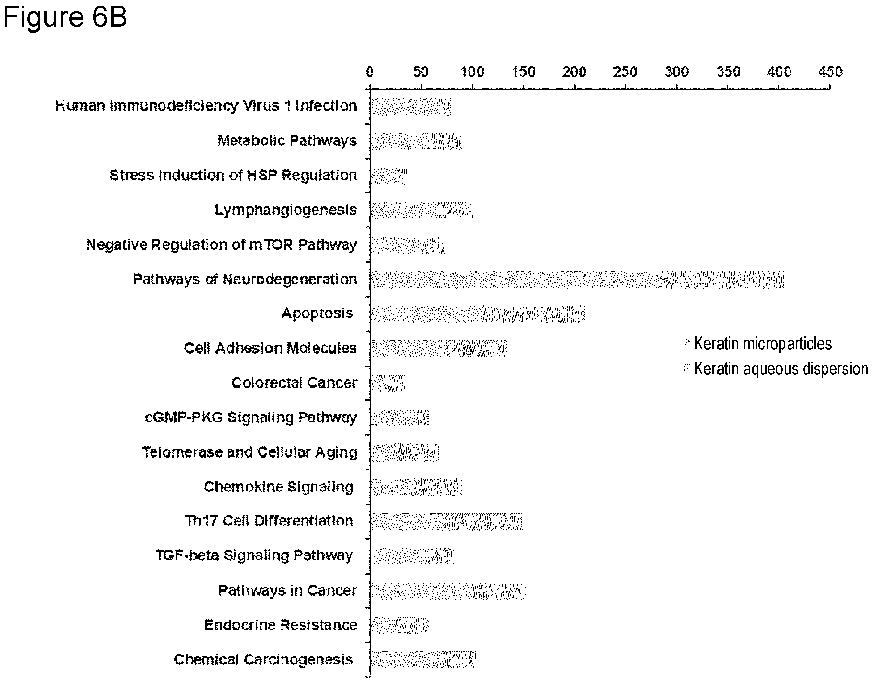

Figure 6A and Figure 6B show the up-regulated (A) or down-regulated (B) gene pathways compared with the control by the treatment with the keratin microparticles or the keratin aqueous dispersion. For example, when compared with the control, the genes of Hallmark of Myc Target, VEGF Signaling Pathway, and Innate Immune System Pathway were up-regulated, whereas the genes of Pathways of Neurodegeneration were down-regulated.

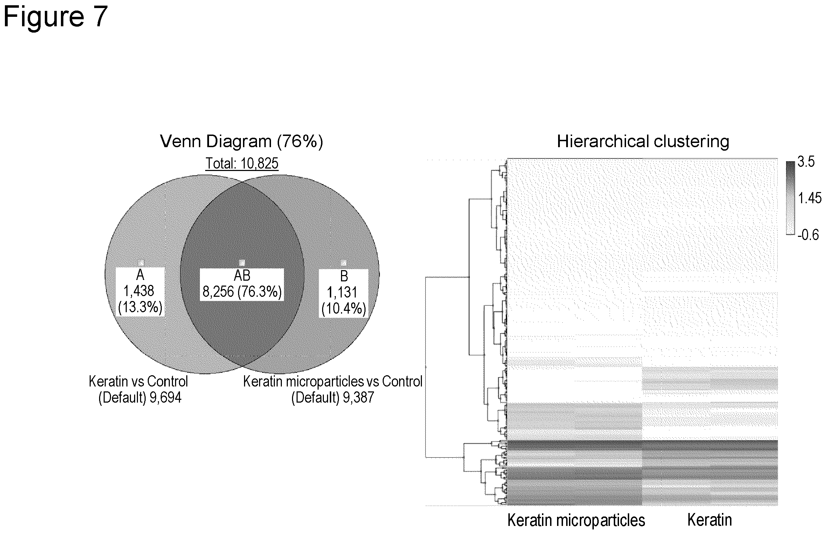

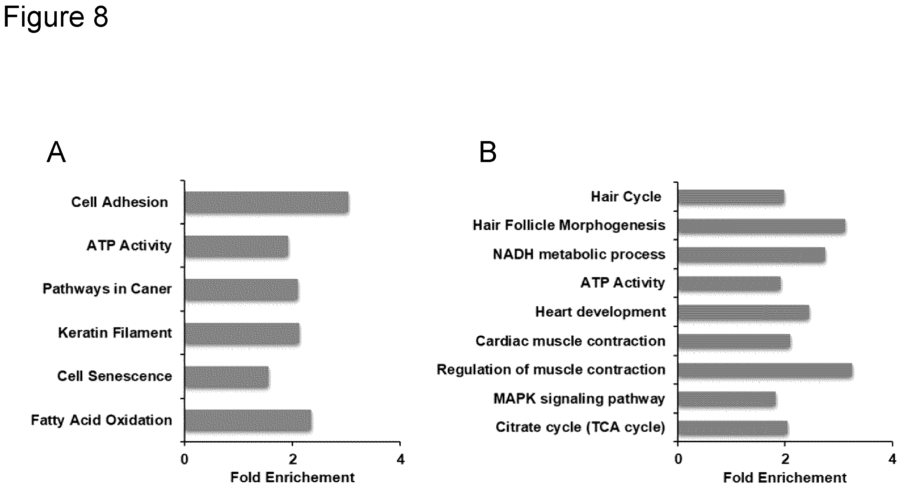

Subsequently, the gene expressions treated with the keratin microparticles and the keratin aqueous dispersion were compared. Of the genes meeting the criteria 1148 among the investigated gene total number 45077, the keratin microparticles application up-regulated 616 genes and down-regulated 532 genes. Figure 7 shows the Venn Diagram and heat map of Hierarchical clustering of the genes that have changed when treated with the 1% keratin aqueous dispersion (keratin) and the 1% keratin microparticle suspension (keratin microparticles). Figure 8 shows fold changes in gene expressions in biological processes. For example, expressions of the genes associated with the processes of Cell Adhesion, Hair Follicle Morphogenesis, and Regulation of muscle contraction remarkably increased. These results show that the keratin microparticle treatment increased the genes associated with the hair growth such as Cell Cycle and Hair Cycle but decreased the genes associated with inflammation and stress, thereby suggesting the mechanism capable of providing hair growing and hair fostering effects with no toxicity or side effects.

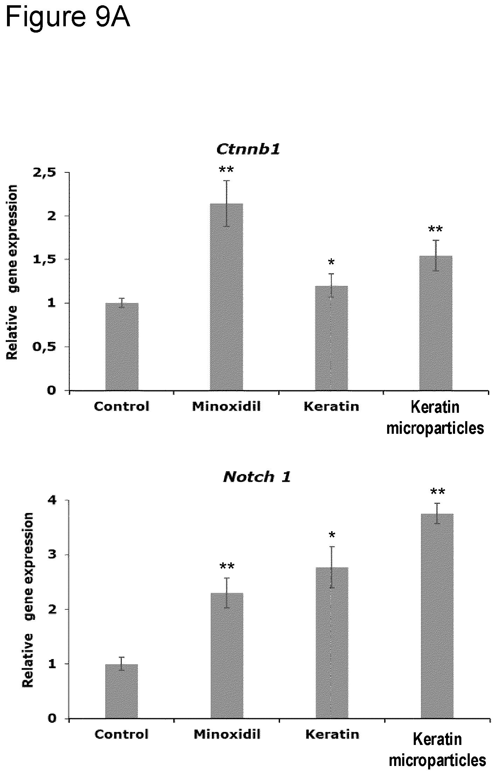

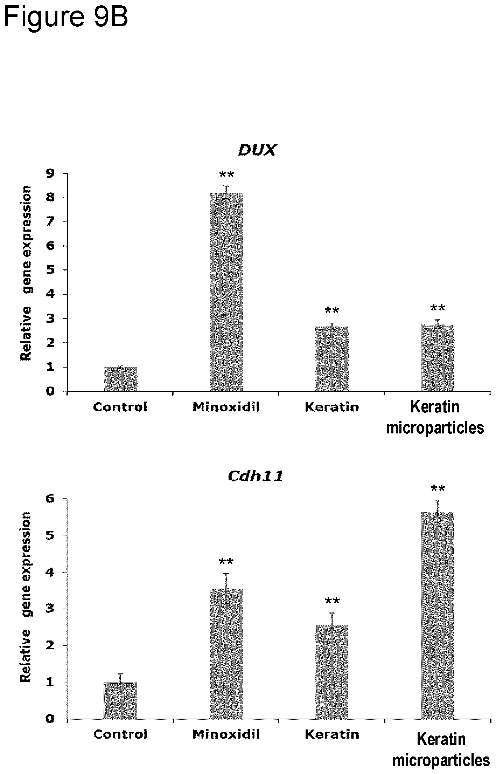

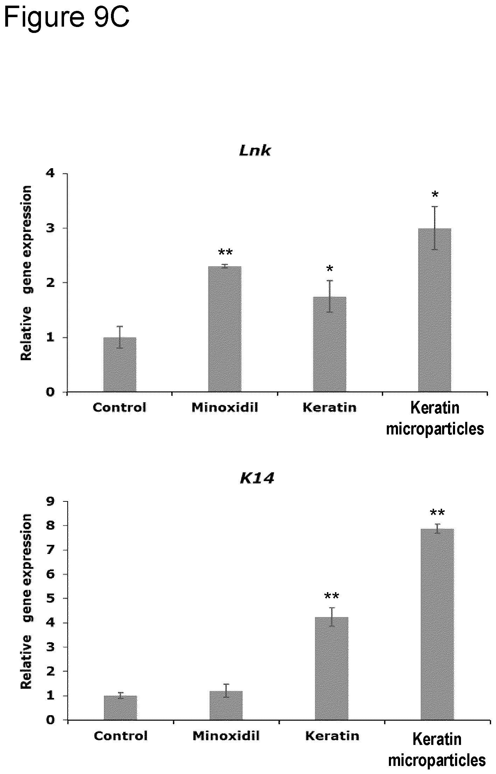

Figure 9A to Figure 9C show the results of microarray validations by RT-PCR. Table 2 below collectively shows the function and change in the expression fold of each gene.

**[Table 2]**

| Gene name | Biological functions | Fold change Keratin aqueous dispersion | Fold change Keratin microparticle |
|---|---|---|---|
| Notch 1 | Hair follicle development, keratinocyte differentiation | 3.38 | 4.29 |
| Ctnnb1 | Melanocyte stem cell differentiation, MITF activation, pigment deposition, hair follicle morphology formation, hair cycle process, positive control of fibroblast growth factor | 1.67 | 2.04 |
| Cadherin | Mesenchymal stem cell differentiation, hair growth cycle, melanocyte and keratinocyte adhesion, keratinocyte differentiation | 2.67 | 5.09 |
| K14 | Control of K14 cell cycle, proliferation, Notch1 and AKT pathways | 2.34 | 6.98 |
| Lnk | Inflammatory disease melanin biosynthesis process by Jak/Stat activation, inhibition; pigment deposition | 1.76 | 2.11 |
| Dux | Mesenchymal stem cell migration, hair follicle stem cell differentiation | 2.06 | 3.02 |

The above results show that the mechanism of keratin microparticles treated group is considered to activate cell adhesion molecules as the target which leads to the activation of mainly hair fostering related pathways. As a result, the activation of hair growth related pathways such as Notch, shh, JAK/STAT and BMP was confirmed. The expressions of all genes were significantly increased compared with the keratin aqueous dispersion treated group.

It was also confirmed that the expressions of genes associated with the migration of epidermal cell and mesenchymal cell increases, the communication of keratinocyte, melanocyte and follicle dermal papilla is promoted, and the hair fostering cycle is further enhanced. Enhanced differentiation ability of epidermal stem cell and keratinocyte, the origins of hair matrix cell, can provide the expectation of further effects on the epidermal stem cell.

Mesenchymal cells such as follicle dermal papilla are present in the dermis part, the deepest part of the skin, and thus the keratin in the keratin aqueous dispersion may be left partially undissolved thereby failing to reach such part. It is considered that these molecules activated the hair growth related genes in the follicle dermal papilla targeting the genes of growth factors and keratin in the epidermis. Additionally, Notch pathway-related genes such as Notch 1, which is known to suppress keratinocyte proliferation and promote the differentiation that rather leads to hair follicle development, are activated, which also further proves the above explanation.

### [Example 4] Application properties of keratin microparticle suspension to skin surface

In the present example, a test was carried out on the adsorption and dry states of a keratin microparticle suspension on a skin surface after applied to the skin surface. Specifically, a fluorescent dye (Acid Red 52) was added to the keratin microparticles (1% suspension) and the keratin aqueous solution (1% suspension) prepared in Example 2 and then each suspension was applied to the surface of a pigskin to investigate the adsorption on the skin surface. Similarly, a 1% aqueous dispersion of keratin powder was applied to the pigskin surface to investigate the adsorption on the skin surface.

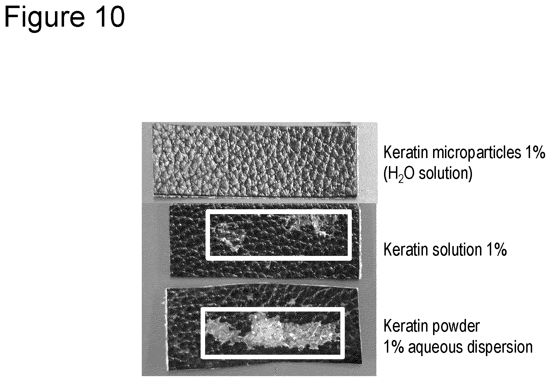

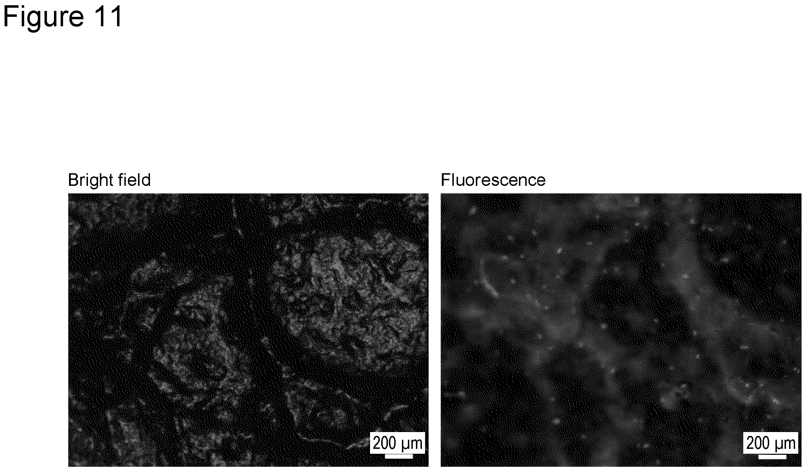

The results are shown in Figures 10 and 11. As shown in Figure 10, it was confirmed that in the case of applying the keratin aqueous solution or the 1% aqueous dispersion of keratin powder, keratin aggregates after dried, whereas in the case of applying the 1% aqueous dispersion of keratin microparticles, keratin microparticles are homogeneously dispersed throughout the entire skin surface. The dispersion of keratin microparticles throughout the entire surface is evident from the fluorescence microscopic images shown in Figure 11.

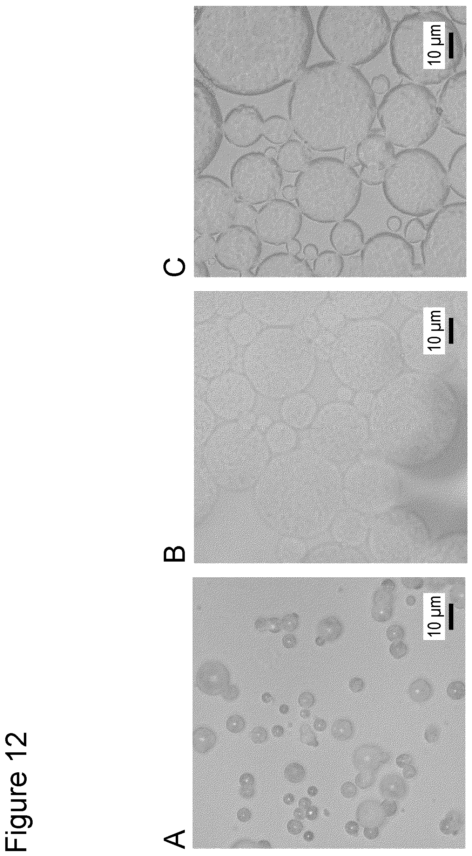

When the suspension of keratin microparticles is applied onto a glass and then naturally dried, a state where disk-like structures cover the surface is observed (Figure 12). As shown in Figure 12, the particle sizes of keratin microparticles (spheres in the dry state) were 1 to 10 µm (A), the particle sizes of keratin microparticles dispersed in water (swollen spherical gel) were 10 to 100 µm (B), and the diameters of keratin microparticles dispersed in water dropped on a glass and dried (dried disk-like structure) were 10 to 100 µm (C). This shows that keratin microparticles were swollen in water and formed spherical gel without being thoroughly dissolved. This leads to the presumption that keratin in water forms a three-dimensional network structure which induces the property similar to the hair growing effect by keratin in the 3D cells.

### [Example 5] Application of keratin microparticle suspension to human subject

Effects of keratin microparticles were investigated when externally applied to a human subject by the following procedure. For a test solution, the 1% keratin microparticle aqueous solution prepared in Example 2 can be used.
(1) Left and right sideburn parts are shaved using an electric shaver and a hair clipper.
(2) The 1% aqueous solution of keratin microparticles is applied, using a cotton swab, to an area of about 3×6 cm including the right sideburn part. The aqueous solution was applied in an amount of about 0.4 mL at one time, twice a day in the morning (after waking up) and at night (after bathing).

As a result of the above test, no side effect such as allergies and rashes were caused after 14 days. Additionally, the applied part at the right sideburn was confirmed to have hair grown to a downward direction when compared with the left sideburn to which the aqueous solution was not applied.

All of the publications, patents and patent publications cited herein are incorporated in their entirety by reference into the present description.

### Industrial Applicability

The present invention provides a hair growing or hair fostering agent exhibiting high hair growing and hair fostering effects. The present invention is applicable in the fields of cosmetic materials, cosmetic products, medicines and the like.

## Claims

1. A hair growing or hair fostering agent comprising keratin microparticles as a hair growing or hair fostering active ingredient.

2. The hair growing or hair fostering agent according to claim 1, wherein the keratin microparticles have particle sizes of 0.01 to 100 µm.

3. The hair growing or hair fostering agent according to claim 1, wherein the keratin microparticles are in the form of an aqueous solution, an aqueous dispersion, or a hydrogel.

4. The hair growing or hair fostering agent according to claim 1, wherein the keratin comprises human keratin, wool-derived keratin, or partially sulfonated or ionized water-soluble keratin.

5. A cosmetic composition comprising the hair growing or hair fostering agent according to claim 1 and a cosmetically acceptable excipient.

6. A pharmaceutical composition comprising the hair growing or hair fostering agent according to claim 1 and a pharmaceutically acceptable excipient.

7. The pharmaceutical composition according to claim 6, which is an external preparation for skin.
